# EUROPEAN PATENT APPLICATION

(11) **EP 2 360 154 A1**
(43) Date of publication of application: **24.08.2011**
(21) Application number: 09769389.9
(22) Date of filing: 25.06.2009
(51) Int. Cl.: C07D 401/00, C07B 59/00, C07C 49/633, C07C 49/443, C07D 303/16

(54) **DEUTERATED VITAMIN D COMPOUNDS**

(30) Priority: 27.06.2008 ES 200801938
(71) Applicant: Universidade De Santiago De Compostela, 15782 Santiago de Compostela (ES)
(72) Inventor: MAESTRO SAAVEDRA, Miguel A., E-15782- Santiago de Compostela (ES); MOURIÑO MOSQUERA, Antonio, E-15782- Santiago de Compostela (ES); NICOLETTI, Daniel, E-15782- Santiago de Compostela (ES); SIGÜEIRO PONTE, Rita, E-15782- Santiago de Compostela (ES)
(74) Representative: ABG Patentes, S.L.
(86) International application number: PCT/ES2009/070251
(87) International publication number: WO 2009/156543

(57) **Abstract**

The invention relates to deuterated vitamin D compounds and the process for preparing and synthesizing a class of vitamin D compounds which can be isotopically substituted. These compounds can be used as internal standards in methods for the quantification of vitamin D, its metabolites and analogues by mass spectrometry. These compounds are described by means of general formula I.

## Description

### Field of the Art

The invention relates to compounds of formula I characterized by being deuterated derivatives of vitamins D₂ and D₃, to processes for obtaining said compounds and to intermediates useful in the synthesis thereof. These compounds of formula I are used as internal standards in methods for the quantification of vitamin D, its metabolites and/or analogues by mass spectrometry.

### State of the Art

The metabolic activation steps and the functions of vitamin D have been discovered as a result of the use of vitamin D metabolites and analogues which incorporated radioactive tracers.

Deuterated vitamin D metabolites and analogues (see H.F. DeLuca et. al, patent US 4,297,289; E.G. Baggiolini et. al, patent US 4,898,855 and E.G. Baggiolini et. al, patent US 5,247,123) are useful as isotopically substituted reference substances of different vitamins D and metabolites for use thereof in methods for the quantification of vitamin D (of its metabolites and/or analogues) in blood plasma and tissues by direct mass spectrometry or by means of series-coupled (tandem) techniques with gas or liquid chromatography.

Vitamins with isotopic labeling (deuterium, tritium) in a single position, C6, are prepared in Patent US 4,297,289.

In patents US 4,898,855 and US 5,247,123, hexa- and octa-deuterated compounds of a single vitamin D₃ metabolite, 1α,25-dihydroxyvitamin D₃, are prepared.

In addition, patent WO 9851678 of S.G. Reddy claims deuterated 3-epivitamins D₃, although its preparation is not specified and the methodology described could be difficult to follow by a person skilled in the art.

The present invention relates to compounds with high degrees of deuteration in vitamin D₃ and D₂ metabolites and analogues, compounds having the side chain characteristic of the vitamin, without hydroxyl in C25, compounds with greater complexity than those known and compounds with epimeric configuration in C3. A methodology with greater synthesis versatility aimed at a broad group of compounds is provided.

### Description of the Invention

In one aspect, the present invention relates to compounds of formula I 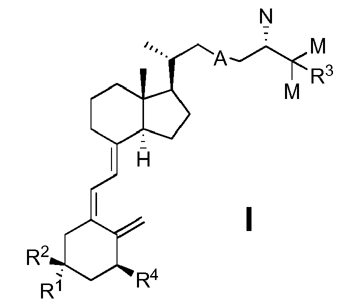 wherein
A is a single, double or triple bond,
M is a trideuteromethyl group,
N is a hydrogen, trideuteromethyl, methyl, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate or acetate groups,
R¹, R², R³ and R⁴ are independently selected from hydrogen, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate or acetate groups,
with the proviso that when N is hydrogen or methyl, then R² is necessarily hydroxyl.

The compounds of formula I are deuterated compounds derived from vitamins D₂ and D₃, used as internal standards in methods for the quantification of vitamin D, its metabolites and/or analogues by mass spectrometry.

In a particular aspect, the invention relates to the following compounds of formula I:
26,26,26,27,27,27,28,28,28-nonadeuterovitamin D₂ (1a), 26,26,26,27,27,27,28,28,28-nonadeutero-25-hydroxyvitamin D₂ (1b),
26,26,26,27,27,27,28,28,28-nonadeutero-1α-hydroxyvitamin D₂ (1c),
26,26,26,27,27,27,28,28,28-nonadeutero-1α,25-dihydroxyvitamin D₂ (1d),
26,26,26,27,27,27-hexadeutero-3-epi-1α-hydroxyvitamin D₃ (1e),
26,26,26,27,27,27-hexadeutero-3-epi-1α,25-dihydroxyvitamin D₃ (1f),
26,26,26,27,27,27-hexadeutero-3-epi-1α-hydroxyvitamin D₂ (1g),
26,26,26,27,27,27,28,28,28-nonadeutero-3-epi-1α-hydroxyvitamin D₂ (1h),
26,26,26,27,27,27-hexadeutero-3-epi-1α,25-dihydroxyvitamin D₂ (1i),
26,26,26,27,27,27,28,28,28-nonadeutero-3-epi-1α,25-dihydroxyvitamin D₂ (1j),
26,26,26,27,27,27-hexadeutero-24R,25-dihydroxyvitamin D₃ (1k), 26,26,26,27,27,27-hexadeutero-1α,24R,25-trihydroxyvitamin D₃ (11).

In another aspect, the invention relates to a process for preparing the compounds of formula I, wherein
A is a single, double or triple bond,
M is a trideuteromethyl group,
N is a hydrogen, trideuteromethyl, methyl, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate or acetate groups,
R¹, R², R³ and R⁴ are independently selected from hydrogen, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate or acetate groups,
comprising a Wittig-Horner reaction between compounds II and III in the presence of a base 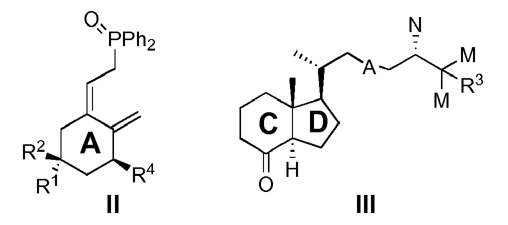 wherein
A is a single, double or triple bond,
M is a trideuteromethyl group,
N is a hydrogen, trideuteromethyl, methyl, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate or acetate groups,
R¹, R², R³ and R⁴ are independently selected from hydrogen, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate or acetate groups.

In another aspect, the invention relates to an alternative process for preparing the compounds of formula I which comprises coupling compounds IV and V in the presence of a catalyst, 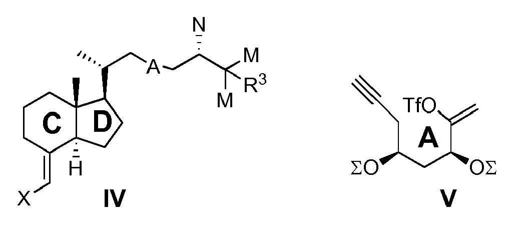 wherein
A is a single, double or triple bond,
M is a trideuteromethyl group,
N is a hydrogen, trideuteromethyl, methyl, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate, or acetate groups,
R³ is selected from hydrogen, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate, or acetate groups,
X is indium bichloride (-InCl2), zinc bromide (-ZnBr) or dialkoxyboron [-B(OR)₂], and
∑ is an SiR₃, SiR₂R', R and R' being alkyl or aryl radicals, or a methoxymethyl group.

In a particular aspect, the catalyst is preferably selected from tetra-kis-triphenylphosphine palladium(0), bis-triphenylphosphine palladium(II) dichloride, bis-(diphenylphosphino)ferrocene palladium(II) dichloride, palladium(II) acetate, bis-acetonitrile palladium(II) dichloride, tris(dibenzylideneacetone)dipalladium(0), nickel(II) acetylacetonate, or bis(1,5-dicyclooctadienyl) nickel(0).

Another aspect of the invention is the intermediate compound of formula III useful in preparing the compounds of formula I 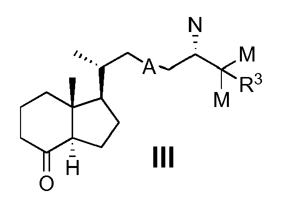 wherein
A is a single, double or triple bond,
M is a trideuteromethyl group,
N is a hydrogen, trideuteromethyl, methyl, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate or acetate groups,
R³ is independently selected from hydrogen, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate or acetate groups,
with the exception of the following compound: 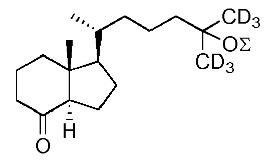 wherein ∑ is an SiR₃, SiR₂R', R and R' being alkyl or aryl radicals, or a methoxymethyl group.

Another aspect of the invention is the intermediate compound of formula IV useful in preparing the compounds of formula I 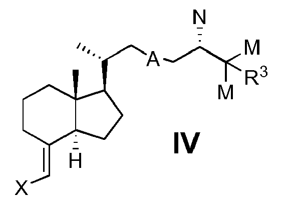 wherein
A is a single, double or triple bond,
M is a trideuteromethyl group,
N is a hydrogen, trideuteromethyl, methyl, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate or acetate groups,
R³ is independently selected from hydrogen, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate or acetate groups,
X is indium bichloride (-InCl₂), zinc bromide (-ZnBr) or dialkoxyboron [-B(OR)₂].

Another aspect of the invention is the compounds of formula VI, VII, VIII, IX, X and XI, intermediates useful in preparing the compounds of formula I

The compounds of formula VI 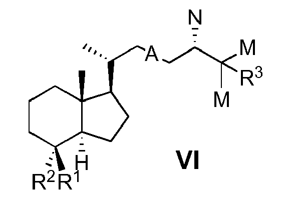 wherein
A is a single, double or triple bond,
M is a trideuteromethyl group,
N is a hydrogen, trideuteromethyl, methyl, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate or acetate groups,
R¹ is a hydroxyl, trialkylsilyloxy or benzoate group,
R² is hydrogen, a hydroxyl group, a trialkylsilyloxy group; or R¹ and R² are both halomethylene,
R³ is selected from hydrogen, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate, or acetate groups,
with the exception of the following compound: 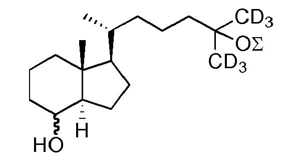 wherein ∑ is an SiR₃, SiR₂R', R and R' being alkyl or aryl radicals, or a methoxymethyl group.

The compounds of formula VII 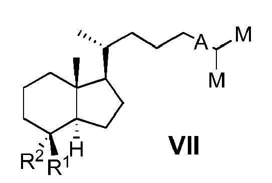 wherein
A is a single or double bond,
M is a trideuteromethyl group,
R¹ is a hydroxyl, trialkylsilyloxy or benzoate group,
R² is hydrogen, or R¹ and R² can both be a halomethylene or carbonyl group.

The compounds of formula VIII 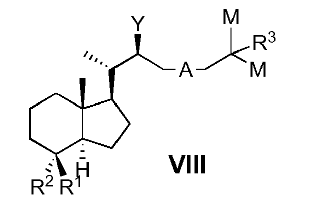 wherein
A is a single, double or triple bond,
M is a trideuteromethyl group,
Y is a hydroxyl, carbamate or diethylphosphate group,
R¹ is a hydroxyl, trialkylsilyloxy or benzoate group,
R² is hydrogen, or R¹ and R² can both be a halomethylene or carbonyl group.

The compounds of formula IX, X, and XI 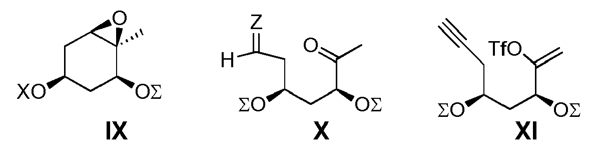 wherein
Z is an oxygen or a dibromomethylene group,
X is a hydrogen, a hydroxyl group or a trialkylsilyloxy group,
∑ is an SiR₃, SiR₂R', R and R' being alkyl or aryl radicals,
or a methoxymethyl group.

### Detailed Description of the Invention

The compounds of formula I are prepared by means of a convergent synthesis through any of the two processes shown in scheme 1.

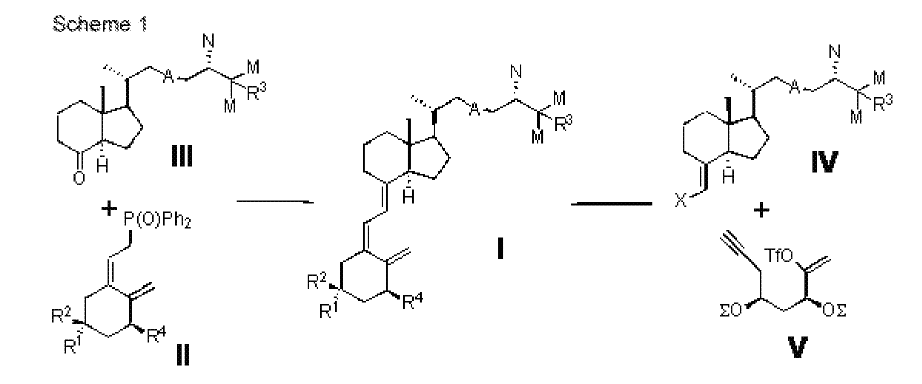

The compounds of formula I are prepared by means of reacting the compounds of formula II with a base and subsequently reacting with the compounds of formula III, wherein, this coupling can be carried out in a particular manner by reacting the anion of the compound of formula II, which is prepared by reacting the compound of formula II with *n-*butyl lithium at -78°C in anhydrous tetrahydrofuran, with compounds of formula III. If necessary, it is possible to carry out the deprotection of the protective groups. This deprotection is preferably performed with tetra-*n*-butylammonium fluoride in tetrahydrofuran at room temperature and with acidic ion-exchange resin AG W50 X4 in deoxygenated methanol.

Alternatively, the compounds of formula I are prepared by means of coupling a compound of formula IV and a compound of formula V in the presence of a catalyst, wherein

In a preferred embodiment, the compounds of formula IV are prepared by reacting the compounds of formula VI, wherein R¹ and R² are both halomethylene, with *t-*butyl lithium at -78°C in anhydrous tetrahydrofuran followed by treatment with a reagent selected from indium trichloride, zinc dibromide, isopropoxycatecholborane or 2-isopropoxy-4,4,5,5-tetramethyl-1,3,2-dioxaborolane.

The coupling is carried out in the presence of a catalyst preferably selected from tetra-*kis*-triphenylphosphine palladium(0), bis-triphenylphosphine palladium(II) dichloride, bis-(diphenylphosphino)ferrocene palladium(II) dichloride, palladium(II) acetate, bis-acetonitrile palladium(II) dichloride, tris(dibenzylideneacetone)dipalladium(0), nickel(II) acetylacetonate, or bis(1,5-dicyclooctadienyl) nickel(0), with a compound of formula V. If necessary, the deprotection of the protective groups is performed in a particular manner with tetra-*n*-butylammonium fluoride in tetrahydrofuran at room temperature and with acidic ion-exchange resin AG W50 X4 in deoxygenated methanol.

### Particular Embodiment of the Invention

The following examples and remarks are provided to allow the persons skilled in the art to better understand and put the invention into practice. They should not be considered as limiting to the scope of the invention, but rather as illustrative and representative examples thereof.

The compounds of formula III can be prepared in a particular manner by a sequence of reactions from the already known compound of formula 2 (Scheme 2). The compound of formula 3 is obtained by stereoselective addition of the anion of protected (1,1,1,2,2,2)-d₆ 2-methyl-3-butyn-2-ol.

The compound of formula 4 is prepared by partial hydrogenation of the triple bond by treatment with hydrogen and the Lindlar catalyst in anhydrous methanol.

The compound of formula 5 is obtained by reaction with phenyl isocyanate in anhydrous pyridine.

The compounds of formula 6 are prepared by substitution reaction with trideuterated or non-deuterated higher order methyl cuprates.

The compounds of formula 7 are obtained by reaction with lithium and aluminium hydride in anhydrous tetrahydrofuran.

The compounds of formula 8 are prepared by oxidation with pyridinium dichromate in anhydrous dichloromethane.

The compounds of formula 9 are prepared through the deprotection sequence, reaction with methyl chloroglyoxylate and deoxygenation by treatment with triphenyl tin hydride in toluene.

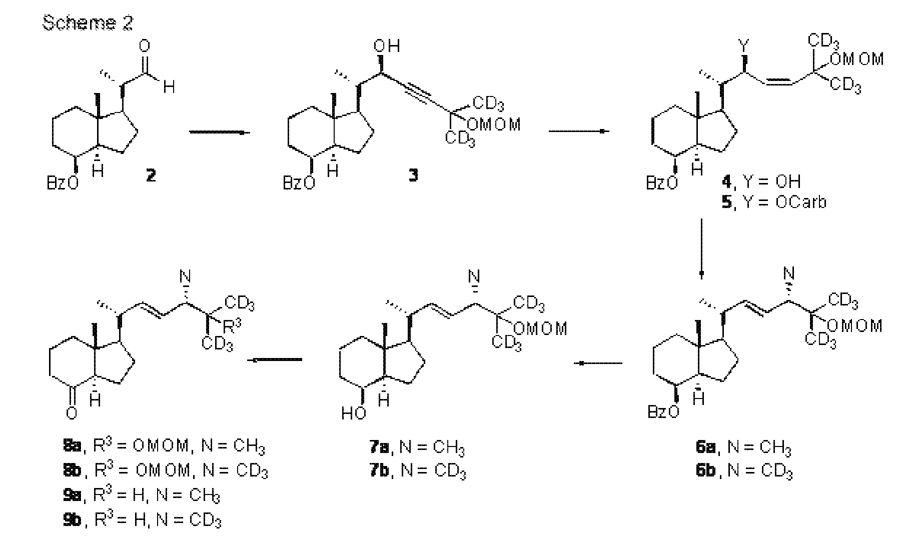

Alternatively, as shown in scheme 3, the compounds of formula III corresponding to the deuterated ketones of the side chain CD bicycle of vitamin D₂ can be prepared in a particular manner by a sequence of reactions from the already known compound of formula 10. The compound of formula 11 is obtained by stereoselective addition of the anion of the protected (1,1,1,2,2,2)-d₆ (*E*)-2-methyl-3-buten-2-ol.

The compound of formula 12 is prepared by reaction with diethyl chlorophosphate in anhydrous pyridine.

The compounds of formula 13 are prepared by substitution reaction with trideuterated or non-deuterated higher order methyl cuprates.

The compounds of formula 7 are obtained by deprotection of the protective group with tetra-*n*-butylammonium fluoride in tetrahydrofuran at room temperature.

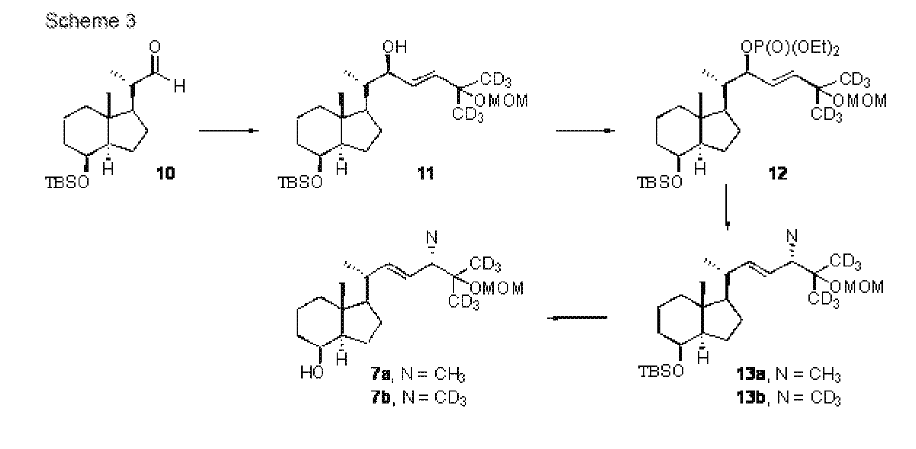

As shown in scheme 4, the compounds of formula III corresponding to the deuterated ketones of the hydroxylated side chain CD bicycle in C-24 are prepared by a sequence of reactions from the compound of formula 11. The compound of formula 14 is obtained by reaction with acetic anhydride in anhydrous pyridine.

The compound of formula 15 is obtained by reaction with bis(acetonitrile)palladium dichloride in anhydrous tetrahydrofuran.

The compound of formula 16 is obtained by hydrogenation with platinum dioxide catalyst in anhydrous methanol.

The compound of formula 17 is obtained by deprotection of the protective group with hydrofluoric acid in tetrahydrofuran:acetonitrile at room temperature.

The compound of formula 18 is obtained by oxidation with pyridinium dichromate in dichloromethane.

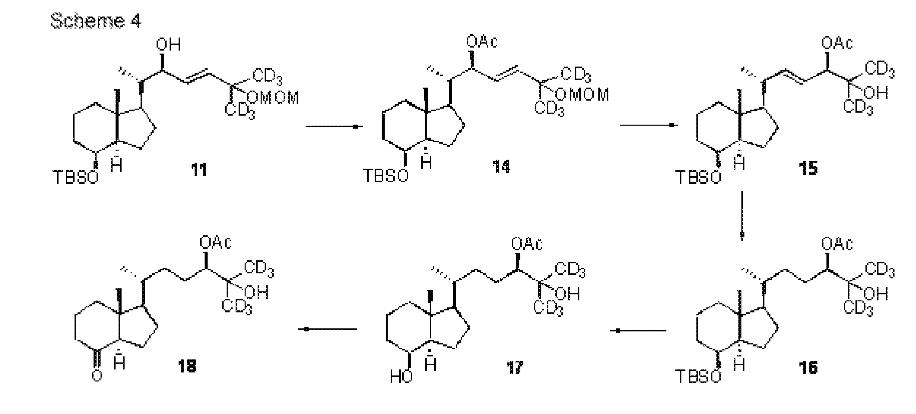

As shown in scheme 5, the compounds of formula III corresponding to the hexadeuterated ketones of the side chain CD bicycle of vitamin D₃ are prepared by a sequence of reactions from the compound of formula 19, which is already known. The compound of formula 20 is prepared by reaction of the compound of formula 19 with *t-*butyl lithium at -78°C in anhydrous THF, addition of copper cyanide and reaction with (1,1,1,2,2,2)-d₆ (*Z*)-2-[2-methyl-3-butene] benzoate.

The compound of formula 21 is prepared by deprotection with tetra-*n*-butylammonium fluoride in anhydrous tetrahydrofuran.

The compound of formula 22 is prepared by hydrogenation with palladium on carbon as a catalyst in methanol.

The compound of formula 23 is prepared by oxidation with pyridinium dichromate in dichloromethane.

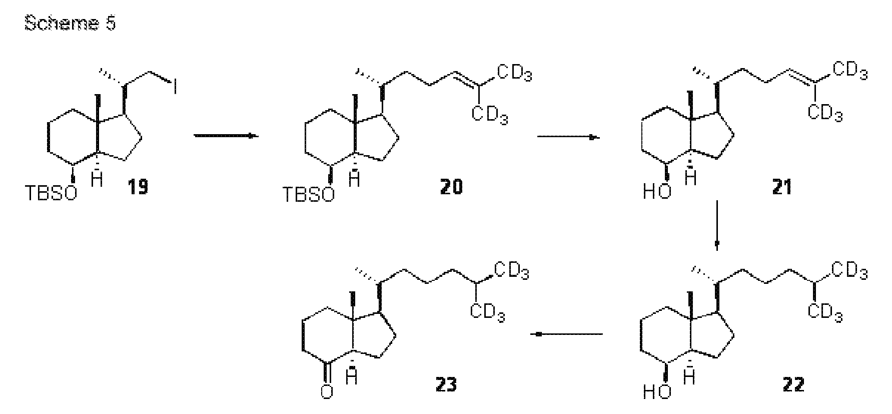

As shown in scheme 6, the compounds of formula V corresponding to the A rings of the 3-epivitamins are prepared by a sequence of reactions from the compound of formula 24, which is already known. The compound of formula 25 is obtained by reaction with periodic acid in anhydrous diethylether.

The compound of formula 26 is prepared by treatment with carbon tetrabromide in dichloromethane.

The compound of formula 27 is prepared by treatment with lithium di-*iso-*propylamide in anhydrous THF followed by trapping enolate with N-(5-chloro-2-pyridyl)triflimide.

The conversion of compound 27 into phosphine oxide 28 required for the Wittig-Horner coupling is performed through known processes.

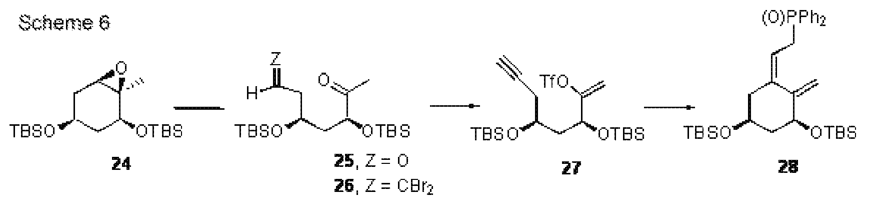

As shown in scheme 7, some of the compounds of formula IV are prepared by a sequence of reactions from the already described compound of formula 8a, 8b, 9a, 9b or 23. The compounds of formula 29, 30, 31, 32, or 33 are obtained by reaction with the anion of bromomethyltriphosphonium bromide in anhydrous toluene.

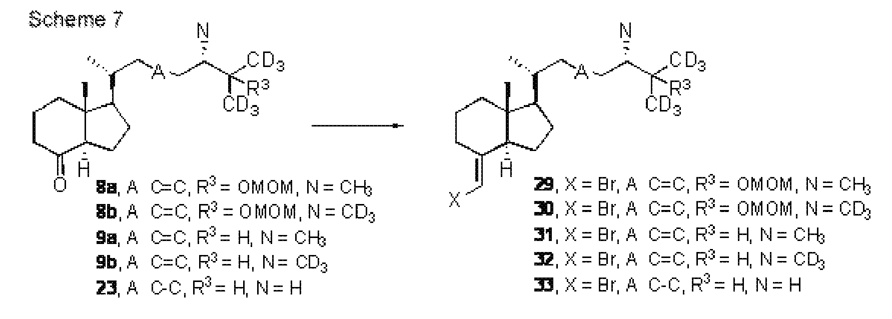

### Example 1

26,26,26,27,27,27,28,28,28-Nonadeuterovitamin D₂ (1a)

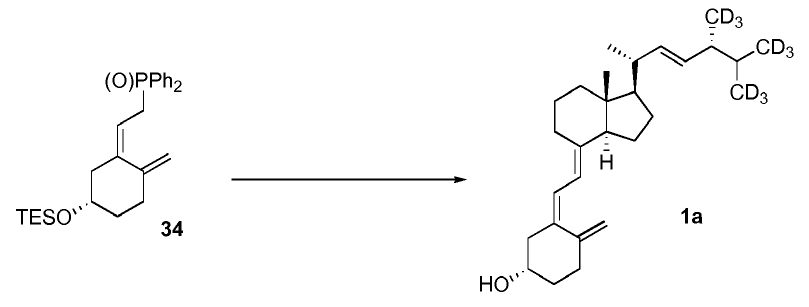

0.21 mmoles of 1.5 M *n-*butyl lithium in hexane are added drop-wise under argon atmosphere to a solution of 0.21 mmoles of phosphine oxide 34 in 5 mL of anhydrous tetrahydrofuran cooled at -78°C. After stirring for 30 minutes, a solution of 0.21 mmoles of ketone 9b in 3 mL of anhydrous tetrahydrofuran is added drop-wise for a period of 30 minutes. The reaction mixture is then stirred at -78°C for 2 hours. The reaction is stopped by adding an aqueous sodium chloride solution and the resulting mixture is left to reach room temperature. The title compound can be isolated by extraction with ethyl acetate and purified by means of flash silica gel column chromatography.

The silicon protective group can be removed by means of treating a solution of the silylated title compound in 2 mL of deoxygenated anhydrous methanol with 200 mg of DOWEX AG 50W-X4 resin under argon atmosphere and stirring in the dark at room temperature for 24 hours. This reaction mixture is stopped by means of adding an aqueous sodium chloride solution and the title compound is isolated by extraction with ethyl acetate and is purified by flash silica gel column chromatography (92% yield).

¹H NMR (250 MHz, CDCl₃, δ) : 6.21 and 6.02 (AB system, 2H, *J* = 12.6 Hz, H-6 and H-7); 5.37 (2H, m, H-22 and H-23); 5.05 (1H, broad s, H-19*E*) ; 4.84 (2H, broad s, H-19*Z*); 3.93 (1H, m, H-3); 1.03 (3H, d, *J* = 7 Hz, CH₃-21); 0.54 (3H, s, CH₃-18)

### Example 2

26,26,26,27,27,27,28,28,28-Nonadeutero-25-hydroxyvitamin D₂ (1b)

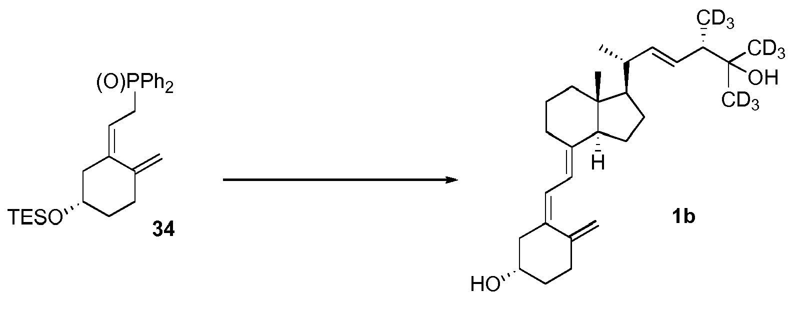

0.55 mmoles of 1.5 M *n-*butyl lithium in hexane are added drop-wise under argon atmosphere to a solution of 0.55 mmoles of phosphine oxide 34 in 10 mL of anhydrous tetrahydrofuran cooled at -78°C. After stirring for 30 minutes, a solution of 0.53 mmoles of ketone 8b in 5 mL of anhydrous tetrahydrofuran is added drop-wise for a period of 30 minutes. The reaction mixture is then stirred at -78°C for 2 hours. The reaction is stopped by adding an aqueous sodium chloride solution. The mixture is left to reach room temperature. The title compound can be isolated by extraction with ethyl acetate and purified by means of flash silica gel column chromatography.

The protective groups can be removed by means of treating a solution of the silylated title compound in 3 mL of deoxygenated anhydrous methanol with 250 mg of DOWEX AG 50W-X4 resin under argon atmosphere and stirring in the dark at room temperature for 24 hours. After adding an aqueous sodium chloride solution, the title compound is isolated by extraction with ethyl acetate and is purified by flash silica gel column chromatography (88% yield).

¹H NMR (250 MHz, CDCl₃, δ) : 6.22 and 6.03 (AB system, 2H, *J* = 12.6 Hz, H-6 and H-7); 5.33 (2H, m, H-22 and H-23); 5.04 (1H, broad s, H-19*E*); 4.84 (2H, broad s, H-19*Z*); 3.95 (1H, m, H-3); 1.03 (3H, d, *J* = 7 Hz, CH₃-21); 0.54 (3H, s, CH₃-18).

### Example 3

26,26,26,27,27,27,28,28,28-Nonadeutero-1α-hydroxyvitamin D₂ (1c)

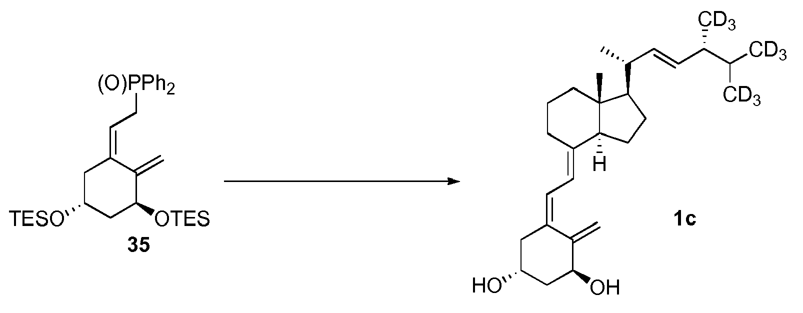

0.25 mmoles of 1.5 M *n-*butyl lithium in hexane are added drop-wise under argon atmosphere to a solution of 0.25 mmoles of phosphine oxide 35 in 6 mL of anhydrous tetrahydrofuran cooled at-78°C. After stirring for 30 minutes, a solution of 0.23 mmoles of ketone 9b in 5 mL of anhydrous tetrahydrofuran is added drop-wise for a period of 30 minutes. The reaction mixture is then stirred at -78°C for 2 hours. The reaction is stopped by adding an aqueous sodium chloride solution. The mixture is left to reach room temperature. The title compound can be isolated by extraction with ethyl acetate and purified by means of flash silica gel column chromatography.

The protective groups can be removed by means of treating a solution of the silylated title compound in 3 mL of deoxygenated anhydrous methanol with 200 mg of DOWEX AG 50W-X4 resin under argon atmosphere and stirring in the dark at room temperature for 24 hours. After adding an aqueous sodium chloride solution, the title compound is isolated by extraction with ethyl acetate and is purified by flash silica gel column chromatography (90% yield).

¹H NMR (250 MHz, CDCl₃, δ) : 6.22 and 6.03 (AB system, 2H, *J* = 12.6 Hz, H-6 and H-7); 5.33 (2H, m, H-22 and H-23); 5.04 (1H, broad s, H-19*E*); 4.84 (2H, broad s, H-19*Z*); 4.43 (1H, m, H-1); 4.29 (1H, m, H-3); 1.03 (3H, d, *J* = 7 Hz, CH₃-21); 0.54 (3H, s, CH₃-18).

### Example 4

26,26,26,27,27,27,28,28,28-Nonadeutero-1α,25-hydroxyvitamin D₂ (1d)

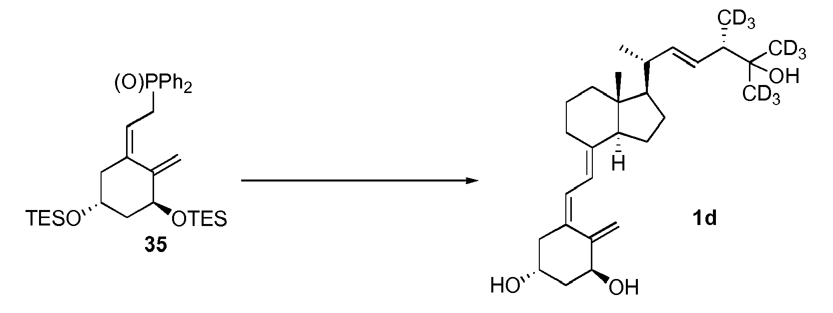

0.21 mmoles of 1.5 M *n-*butyl lithium in hexane are added drop-wise under argon atmosphere to a solution of 0.21 mmoles of phosphine oxide 35 in 5 mL of anhydrous tetrahydrofuran cooled at -78°C. After stirring for 30 minutes, a solution of 0.21 mmoles of ketone 8b in 3 mL of anhydrous tetrahydrofuran is added drop-wise for a period of 30 minutes. The reaction mixture is then stirred at -78°C for 2 hours. The reaction is stopped by adding an aqueous sodium chloride solution and the mixture obtained is left to reach room temperature. The title compound can be isolated by extraction with ethyl acetate and purified by means of flash silica gel column chromatography.

The protective groups can be removed by means of treating a solution of the silylated title compound in 5 mL of deoxygenated anhydrous methanol with 150 mg of DOWEX AG 50W-X4 resin under argon atmosphere and stirring in the dark at room temperature for 24 hours. After adding an aqueous sodium chloride solution, the title compound is isolated by extraction with ethyl acetate and is purified by flash silica gel column chromatography (85% yield).

¹H NMR (250 MHz, CDCl₃, δ) : 6.38 and 6.01 (AB system, 2H, *J* = 11 Hz, H-6 and H-7); 5.35 (2H, m, H-22 and H-23); 5.31 (1H, broad s, H-19*E*); 5.00 (1H, broad s, H-19*Z*); 4.44 (1H, m, H-1); 4.27 (1H, m, H-3); 1.03 (3H, d, *J* = 7 Hz, CH₃-21); 0.55 (3H, s, CH₃-18).

### Example 5

26,26,26,27,27,27-Hexadeutero-3-epi-1α-hydroxyvitamin D₃ (1e)

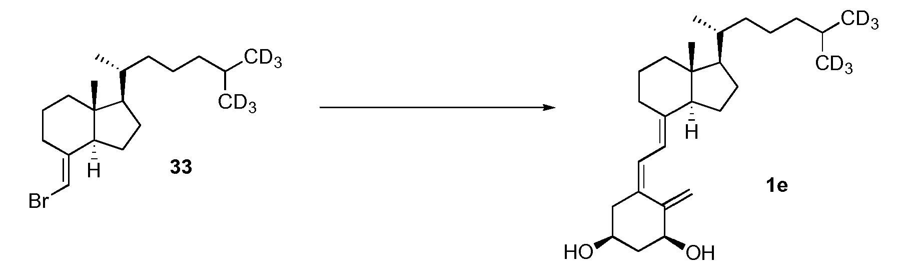

A 0.27 M solution of indium trichloride in THF is added to a solution of 0.43 mmoles of vinyl bromide 33 in 5 mL of anhydrous tetrahydrofuran with stirring and under argon at room temperature. The resulting solution is cooled at -78°C and 1.61 mmoles of 1.5 M *t-*butyl lithium in pentane are added to it drop-wise. After stirring for 1 hour at -78°C and at 0°C for another hour, a suspension of 0.22 mmoles of the enol triflate 27, 0.6 mL of triethylamine, 0.01 mmoles of tetra-kis-(triphenylphosphine) palladium(0) in 5 mL of THF is added. After 3 hours of stirring, the reaction is stopped by adding 3 mL of water. The title compound can be isolated by extraction with hexane and purified by means of flash silica gel column chromatography.

The protective groups are removed by treating a solution of the silylated title compound in 3 mL of anhydrous acetonitrile, 1 mL of dichloromethane and 1 mL of triethylamine, with 0.2 mL of pyridinium fluoride (70% HF/ 30% pyridine) under argon and stirring in the dark at room temperature for 4 hours. After adding an aqueous sodium bicarbonate solution, the title compound is isolated by extraction with ethyl acetate and is purified by flash silica gel column chromatography (72% yield).

¹H NMR (250 MHz, CDCl₃, δ) : 6.43 and 6.01 (AB system, 2H, *J* = 11 Hz, H-6 and H-7); 5.29 (1H, broad s, H-19*E*); 4.99 (1H, broad s, H-19*Z*); 4.31 (1H, m, H-1); 4.05 (1H, m, H-3); 0.93 (3H, d, *J* = 6 Hz, CH₃-21); 0.53 (3H, s, CH₃-18).

### Example 6

26,26,26,27,27,27-Hexadeutero-3-epi-1α,25-dihydroxyvitamin D₃ (1f)

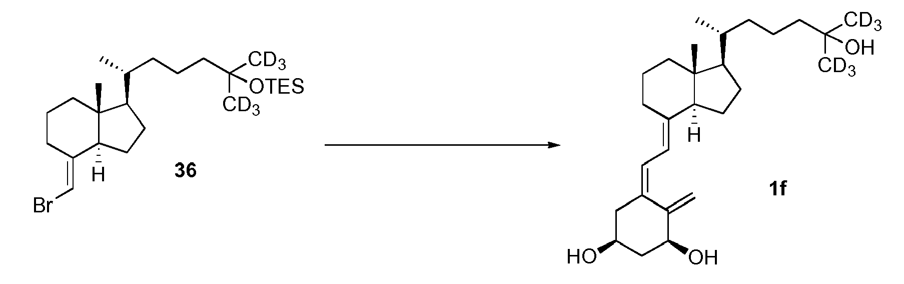

A 0.46 M solution of zinc bromide in THF is added to a solution of 0.21 mmoles of vinyl bromide 36 in 2 mL of anhydrous tetrahydrofuran with stirring and under argon at room temperature. The resulting solution is cooled at -78°C and 0.79 mmoles of 1.5 M *t-*butyl lithium in pentane are added to it drop-wise. After stirring for 1 hour at -78°C and at 0°C for another hour, a suspension of 0.11 mmoles of the enol triflate 27, 0.3 mL of triethylamine, 0.005 mmoles of tetra-kis-(triphenylphosphine) palladium(0) in 2 mL of THF is added. After 3 hours of stirring, the reaction is stopped by adding 3 mL of water. The title compound can be isolated by extraction with hexane and purified by means of flash silica gel column chromatography.

The protective groups are removed by treating a solution of the silylated title compound in 1 mL of anhydrous acetonitrile, 0.5 mL of dichloromethane and 0.5 mL of triethylamine, with 0.1 mL of pyridinium fluoride(70% HF/ 30% pyridine) under argon and stirring in the dark at room temperature for 4 hours. After adding an aqueous sodium bicarbonate solution, the title compound is isolated by extraction with ethyl acetate and is purified by flash silica gel column chromatography (75% yield).

¹H NMR (250 MHz, CDCl₃, δ) : 6.43 and 6.01 (AB system, 2H, *J* = 11 Hz, H-6 and H-7); 5.29 (1H, broad s, H-19*E*); 4.99 (1H, broad s, H-19*Z*); 4.31 (1H, m, H-1); 4.05 (1H, m, H-3); 0.93 (3H, d, *J* = 6 Hz, CH₃-21); 0.53 (3H, s, CH₃-18).

### Example 7

26,26,26,27,27,27-Hexadeutero-3-epi-1α-hydroxyvitamin D₂ (1g)

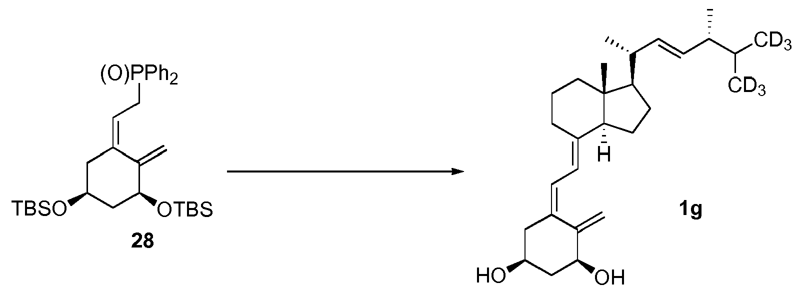

1.85 mmoles of 2.56 M *n-*butyl lithium in hexane are added drop-wise under argon atmosphere to a solution of 1.85 mmoles of phosphine oxide 28 in 30 mL of anhydrous tetrahydrofuran cooled at-78°C. After stirring for 30 minutes, a solution of 0.21 mmoles of ketone 9a in 3 mL of anhydrous tetrahydrofuran is added drop-wise for a period of 30 minutes. The reaction mixture is then stirred at -78°C for 4 hours. The reaction is stopped by adding an aqueous sodium chloride solution and the resulting mixture is left to reach room temperature. The title compound can be isolated by extraction with ethyl acetate and purified by means of flash silica gel column chromatography.

The protective groups are removed by treating a solution of the silylated title compound in 5 mL of anhydrous acetonitrile, 2.5 mL of dichloromethane and 2.5 mL of triethylamine, with 0.5 mL of pyridinium fluoride(70% HF/ 30% pyridine) under argon and stirring in the dark at room temperature for 4 hours. After adding an aqueous sodium bicarbonate solution, the title compound is isolated by extraction with ethyl acetate and is purified by flash silica gel column chromatography (85% yield).

¹H NMR (250 MHz, CDCl₃, δ) : 6.22 and 6.05 (AB system, 2H, *J* = 11 Hz, H-6 and H-7); 5.43-5.23 (2H, m, H-22 and H-23); 5.05 (1H, broad s, H-19*E*); 4.82 (2H, broad s, H-19*Z*); 4.32 (1H, m, H-1); 4.04 (1H, m, H-3); 3.95 (1H, m, H-3); 1.03 (3H, d, *J* = 7 Hz, CH₃-21); 0.54 (3H, s, CH₃-18).

### Example 8

26,26,26,27,27,27,28,28,28-Nonadeutero-3-epi-1α-hydroxyvitamin D₂ (1h)

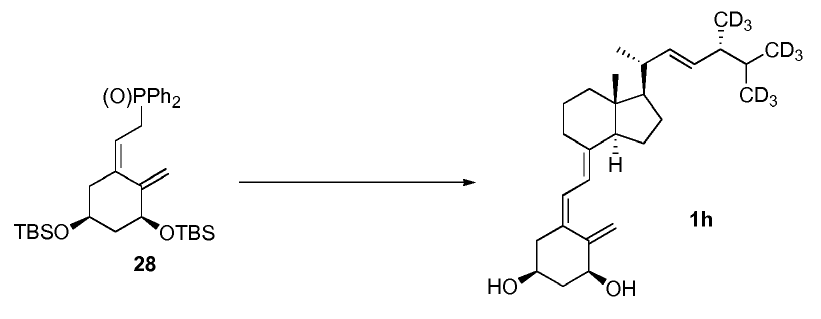

0.093 mmoles of 1.5 M *n-*butyl lithium in hexane are added drop-wise under argon atmosphere to a solution of 0.074 mmoles of phosphine oxide 28 in 2 mL of anhydrous tetrahydrofuran cooled at -78°C. After stirring for 30 minutes, a solution of 0.074 mmoles of ketone 9b in 1 mL of anhydrous tetrahydrofuran is added drop-wise for a period of 30 minutes. The reaction mixture is then stirred at-78°C for 4 hours. The reaction is stopped by adding an aqueous sodium chloride solution and the resulting mixture is left to reach room temperature. The title compound can be isolated by extraction with ethyl acetate and purified by means of flash silica gel column chromatography.

The silicon protective group can be removed by means of treating a solution of the silylated title compound in 2 mL of deoxygenated anhydrous methanol with 200 mg of DOWEX AG 50W-X4 resin under argon atmosphere and stirring in the dark at room temperature for 24 hours. This reaction mixture is stopped by means of adding an aqueous sodium chloride solution and the title compound is isolated by extraction with ethyl acetate and is purified by flash silica gel column chromatography (93% yield).

¹H NMR (250 MHz, CDCl₃, δ) : 6.22 and 6.05 (AB system, 2H, *J* = 11 Hz, H-6 and H-7); 5.43-5.23 (2H, m, H-22 and H-23); 5.05 (1H, broad s, H-19*E*); 4.82 (2H, broad s, H-19*Z*); 4.31 (1H, m, H-1); 4.05 (1H, m, H-3); 3.93 (1H, m, H-3); 0.57 (3H, s, CH₃-18).

### Example 9

26,26,26,27,27,27-Hexadeutero-3-epi-1α,25-dihydroxyvitamin D₂ (1i)

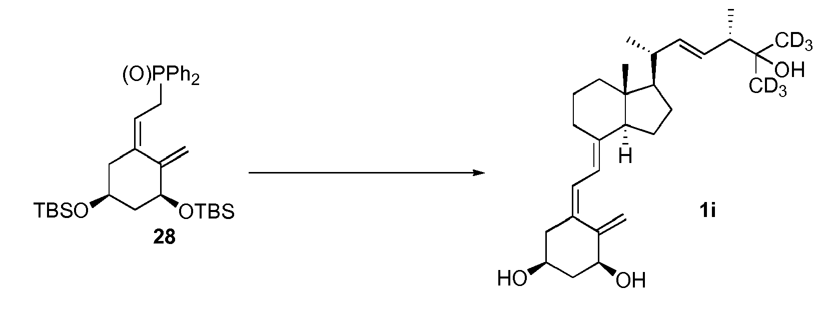

1.73 mmoles of 2.5 M *n-*butyl lithium in hexane are added drop-wise under argon atmosphere to a solution of 1.65 mmoles of phosphine oxide 28 in 20 mL of anhydrous tetrahydrofuran cooled at -78°C. After stirring for 30 minutes, a solution of 0.21 mmoles of ketone 8a in 8 mL of anhydrous tetrahydrofuran is added drop-wise for a period of 30 minutes. The reaction mixture is then stirred at-78°C for 4 hours. The reaction is stopped by adding an aqueous sodium chloride solution and the resulting mixture is left to reach room temperature. The title compound can be isolated by extraction with ethyl acetate and purified by means of flash silica gel column chromatography.

The silicon protective group can be removed by means of treating a solution of the silylated title compound in 10 mL of deoxygenated anhydrous methanol with 650 mg of DOWEX AG 50W-X4 resin under argon atmosphere and stirring in the dark at room temperature for 24 hours. This reaction mixture is stopped by means of adding an aqueous sodium chloride solution and the title compound is isolated by extraction with ethyl acetate and is purified by flash silica gel column chromatography (70% yield).

¹H NMR (250 MHz, CDCl₃, δ) : 6.22 and 6.05 (AB system, 2H, *J* = 11 Hz, H-6 and H-7); 5.43-5.23 (2H, m, H-22 and H-23); 5.05 (1H, broad s, H-19*E*); 4.82 (2H, broad s, H-19*Z*); 4.32 (1H, m, H-1); 4.04 (1H, m, H-3); 3.95 (1H, m, H-3); 1.02 (3H, d, *J* = 7 Hz, CH₃-21); 0.54 (3H, s, CH₃-18).

### Example 10

26,26,26,27,27,27,28,28,28-Nonadeutero-3-epi-1α,25-dihydroxyvitamin D₂ (1j)

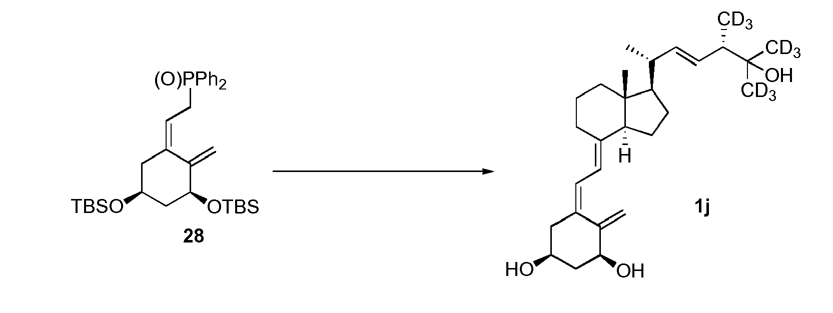

0.50 mmoles of 2.25 M *n-*butyl lithium in hexane are added drop-wise under argon atmosphere to a solution of 0.558 mmoles of phosphine oxide 28 in 9 mL of anhydrous tetrahydrofuran cooled at -78°C. After stirring for 30 minutes, a solution of 0.267 mmoles of ketone 8b in 5 mL of anhydrous tetrahydrofuran is added drop-wise for a period of 30 minutes. The reaction mixture is then stirred at -78°C for 4 hours. The reaction is stopped by adding an aqueous sodium chloride solution and the resulting mixture is left to reach room temperature. The title compound can be isolated by extraction with ethyl acetate and purified by means of flash silica gel column chromatography.

The silicon protective group can be removed by means of treating a solution of the silylated title compound in 10 mL of deoxygenated anhydrous methanol with 650 mg of DOWEX AG 50W-X4 resin under argon atmosphere and stirring in the dark at room temperature for 24 hours. This reaction mixture is stopped by means of adding an aqueous sodium chloride solution and the title compound is isolated by extraction with ethyl acetate and is purified by flash silica gel column chromatography (91% yield).

¹H NMR (250 MHz, CDCl₃, δ) : 6.23 and 6.04 (AB system, 2H, *J* = 11 Hz, H-6 and H-7); 5.45-5.25 (2H, m, H-22 and H-23); 5.05 (1H, broad s, H-19*E*); 4.82 (2H, broad s, H-19*Z*); 4.32 (1H, m, H-1); 4.04 (1H, m, H-3); 3.95 (1H, m, H-3); 1.03 (3H, d, *J* = 7 Hz, CH₃-21); 0.54 (3H, s, CH₃-18).

### Example 11

26,26,26,27,27,27-Hexadeutero-24R,25-dihydroxyvitamin D₃ (1k)

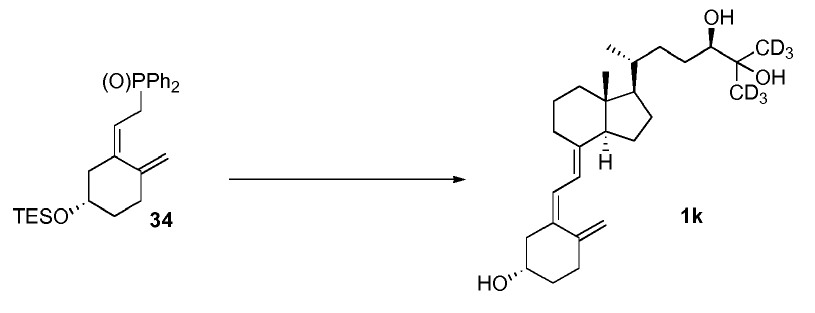

0.90 mmoles of 1.5 M *n-*butyl lithium in hexane are added drop-wise under argon atmosphere to a solution of 0.90 mmoles of phosphine oxide 34 in 20 mL of anhydrous tetrahydrofuran cooled at -78°C. After stirring for 30 minutes, a solution of 0.40 mmoles of ketone 18 in 10 mL of anhydrous tetrahydrofuran is added drop-wise for a period of 30 minutes. The reaction mixture is then stirred at -78°C for 2 hours. The mixture is obtained after adding an aqueous sodium chloride solution. The mixture is left to reach room temperature. The title compound can be isolated by extraction with ethyl acetate and purified by means of flash silica gel column chromatography.

The protective groups can be removed by means of treating a solution of the silylated title compound in 20 mL of anhydrous tetrahydrofuran with 1.5 mmoles of a 1 M solution of tetrabutylammonium fluoride in tetrahydrofuran under argon atmosphere and stirring in the dark at room temperature for 4 hours. The reaction is stopped by means of adding an aqueous sodium chloride solution and the solute is isolated by extraction with ethyl acetate. It is then dissolved in 10 mL of tetrahydrofuran and 1 mmol of a 1M aqueous lithium hydroxide solution is added stirring under argon atmosphere and in the dark at room temperature. After 6 hours, an additional 1 mmol of lithium hydroxide (1M, aq.) is added and the operation is repeated after another 6 hours. After stirring for 24 hours, the reaction mixture is stopped by means of adding an aqueous ammonium chloride solution and the title compound is isolated by extraction with ethyl acetate and is purified by means of flash silica gel column chromatography.

¹H NMR (250 MHz, CDCl₃, δ) : 6.21 and 6.02 (AB system, 2H, *J* = 11 Hz, H-6 and H-7); 5.03 (1H, broad s, H-19*E*); 4.81 (1H, broad s, H-19*Z*); 3.93 (1H, m, H-3); 3.32 (1H, m, H-24); 0.93 (3H, d, *J* = 6 Hz, CH₃-21); 0.54 (3H, s, CH₃-18).

### Example 12

26,26,26,27,27,27-Hexadeutero-1α,24R,25-trihydroxyvitamin D₃ (11)

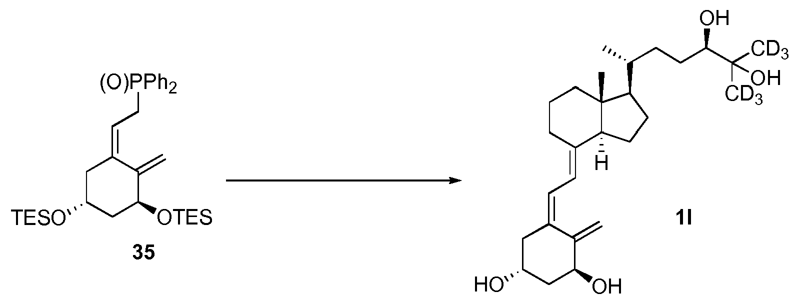

0.65 mmoles of 1.5 M *n-*butyl lithium in hexane are added drop-wise under argon atmosphere to a solution of 0.65 mmoles of phosphine oxide 35 in 15 mL of anhydrous tetrahydrofuran cooled at -78°C. After stirring for 30 minutes, a solution of 0.31 mmoles of ketone 18 in 7 mL of anhydrous tetrahydrofuran is added drop-wise for a period of 30 minutes. The reaction mixture is then stirred at -78°C for 2 hours. The reaction is stopped by adding an aqueous sodium chloride solution and is left to reach room temperature. The title compound can be isolated by extraction with ethyl acetate and purified by means of flash silica gel column chromatography.

The protective groups can be removed by means of treating a solution of the silylated title compound in 20 mL of anhydrous tetrahydrofuran with 1.5 mmoles of a 1M solution of tetrabutylammonium fluoride in tetrahydrofuran under argon atmosphere and stirring in the dark at room temperature for 4 hours. The compound obtained after adding an aqueous sodium chloride solution and extracted with ethyl acetate is dissolved in 10 mL of tetrahydrofuran and 1 mmol of a 1M aqueous lithium hydroxide solution is added stirring under argon atmosphere and in the dark at room temperature. After 6 hours, 1 mmol of a 1M aqueous lithium hydroxide solution is added and the operation is repeated after another 6 hours. After stirring for 24 hours, an aqueous ammonium chloride solution is added and the title compound is isolated by extraction with ethyl acetate and is purified by means of flash silica gel column chromatography.

¹H NMR (250 MHz, CDCl₃, δ) : 6.37 and 6.05 (AB system, 2H, *J* = 12 Hz, H-6 and H-7); 5.33 (1H, broad s, H-19*E*); 5.00 (1H, broad s, H-19*Z*); 4.43 (1H, m, H-1); 4.23 (1H, m, H-3); 3.35 (1H, m, H-24); 0.93 (3H, d, *J* = 6 Hz, CH₃-21); 0.54 (3H, s, CH₃-18).

## Claims

1. A compound of formula I: 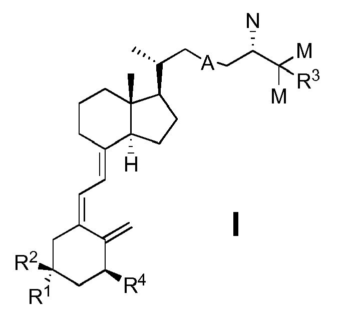 wherein
A is a single, double or triple bond,
M is a trideuteromethyl group,
N is a hydrogen, trideuteromethyl, methyl, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate, or acetate groups, R¹, R², R³ and R⁴ are independently selected from hydrogen, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate, or acetate groups,
with the proviso that when N is hydrogen or methyl, then R² is necessarily hydroxyl.

2. A compound of formula I, selected from
26,26,26,27,27,27,28,28,28-nonadeuterovitamin D₂ (1a), 26,26,26,27,27,27,28,28,28-nonadeutero-25-hydroxyvitamin D₂ (1b),
26,26,26,27,27,27,28,28,28-nonadeutero-1α-hydroxyvitamin D₂ (1c),
26,26,26,27,27,27,28,28,28-nonadeutero-1α,25-dihydroxyvitamin D₂ (1d),
26,26,26,27,27,27-hexadeutero-3-epi-1α-hydroxyvitamin D₃ (1e),
26,26,26,27,27,27-hexadeutero-3-epi-1α,25-dihydroxyvitamin D₃ (1f),
26,26,26,27,27,27-hexadeutero-3-epi-1α-hydroxyvitamin D₂ (1g),
26,26,26,27,27,27,28,28,28-nonadeutero-3-epi-1α-hydroxyvitamin D₂ (1h),
26,26,26,27,27,27-hexadeutero-3-epi-1α,25-dihydroxyvitamin D₂ (1i),
26,26,26,27,27,27,28,28,28-nonadeutero-3-epi-1α,25-dihydroxyvitamin D₂ (1j),
26,26,26,27,27,27-hexadeutero-*24R*,25-dihydroxyvitamin D₃ (1k), 26,26,26,27,27,27-hexadeutero-1α,*24R*,25-trihydroxyvitamin D₃ (11).

3. A process for preparing the compounds of formula I, 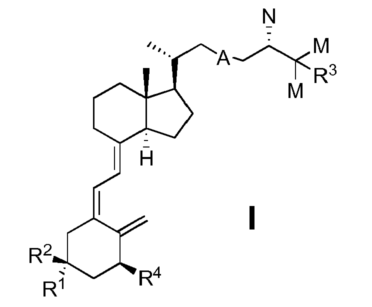 wherein
A is a single, double or triple bond,
M is a trideuteromethyl group,
N is a hydrogen, trideuteromethyl, methyl, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate, or acetate groups,
R¹, R², R³ and R⁴ are independently selected from hydrogen,
hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate, or acetate groups, comprising a Wittig-Horner reaction between compounds II and III in the presence of a base. 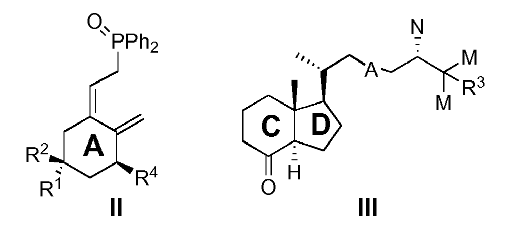 wherein
A is a single, double or triple bond,
M is a trideuteromethyl group,
N is a hydrogen, trideuteromethyl, methyl, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate or acetate groups, R¹, R², R³ and R⁴ are independently selected from hydrogen, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate or acetate groups.

4. The process according to claim 3, **characterized in that** the base used is preferably selected from *n-*butyl lithium, potassium *t-*butoxide, sodium hydride or sodium amide.

5. A process for preparing the compounds of formula I, 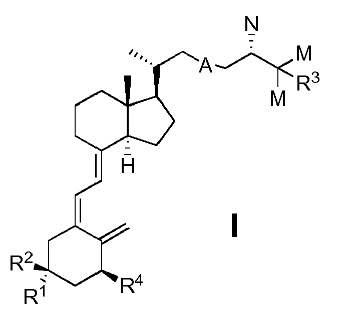 wherein
A is a single, double or triple bond,
M is a trideuteromethyl group,
N is a hydrogen, trideuteromethyl, methyl, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate, or acetate groups, R¹, R², R³ and R⁴ are independently selected from hydrogen, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate, or acetate groups,
which comprises coupling compounds IV and V in the presence of a catalyst, 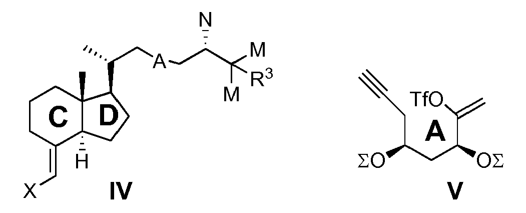 wherein
A is a single, double or triple bond,
M is a trideuteromethyl group,
N is a hydrogen, trideuteromethyl, methyl, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate or acetate groups,
R³ is selected from hydrogen, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate or acetate groups,
X is indium bichloride (-InCl₂), zinc bromide (-ZnBr) or dialkoxyboron [-B(OR)₂], and
∑ is an SiR₃, SiR₂R', R and R' being alkyl or aryl radicals, or a methoxymethyl group.

6. The process according to claim 5, wherein the catalyst used is preferably selected from tetra-*kis*-triphenylphosphine palladium(0), bis-triphenylphosphine palladium(II) dichloride, bis-(diphenylphosphino)ferrocene palladium(II) dichloride, palladium(II) acetate, bis-acetonitrile palladium(II) dichloride, tris(dibenzylideneacetone)dipalladium(0), nickel(II) acetylacetonate or bis(1,5-dicyclooctadienyl) nickel(0).

7. The compounds of formula III, 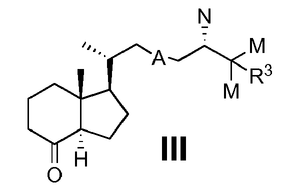 wherein
A is a single, double or triple bond,
M is a trideuteromethyl group,
N is a hydrogen, trideuteromethyl, methyl, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate or acetate groups,
R³ is independently selected from hydrogen, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate or acetate groups,
with the exception of the following compound: 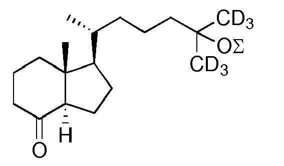 wherein ∑ is an SiR₃, SiR₂R', R and R' being alkyl or aryl radicals, or a methoxymethyl group.

8. The compounds of formula IV, 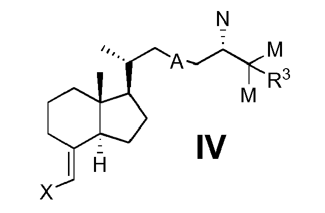 wherein
A is a single, double or triple bond,
M is a trideuteromethyl group,
N is a hydrogen, trideuteromethyl, methyl, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate or acetate groups,
R³ is independently selected from hydrogen, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate or acetate groups,
X is indium bichloride (-InCl₂), zinc bromide (-ZnBr) or
dialkoxyboron [-B(OR)₂].

9. The compounds of formula VI, 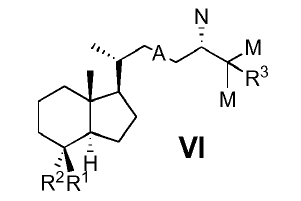 wherein
A is a single, double or triple bond,
M is a trideuteromethyl group,
N is a hydrogen, trideuteromethyl, methyl, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate or acetate groups,
R¹ is a hydroxyl, trialkylsilyloxy or benzoate group,
R² is hydrogen, a hydroxyl group, a trialkylsilyloxy group; or R¹ and R² are both halomethylene,
R³ is selected from hydrogen, hydroxyl or -OR, wherein R is selected from methoxymethyl, methoxyethyl, trimethylsilylethoxymethyl, trimethylsilyl, triethylsilyl, *t-*butyldimethylsilyl, tri-*iso-*propylsilyl, *t-*butyldiphenylsilyl, dimethylphenylsilyl, dimethylbenzylsilyl, benzoate, *p-*nitrobenzoate, pivalate or acetate groups,
with the exception of the following compound: 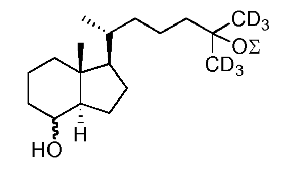 wherein ∑ is an SiR₃, SiR₂R', R and R' being alkyl or aryl radicals, or a methoxymethyl group.

10. The compounds of formula VII, 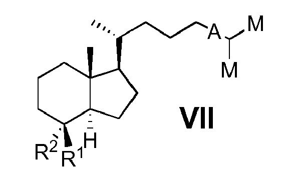 wherein
A is a single or double bond,
M is a trideuteromethyl group,
R¹ is a hydroxyl, trialkylsilyloxy or benzoate group,
R² is hydrogen, or R¹ and R² can both be a halomethylene or carbonyl group.

11. The compounds of formula VIII, 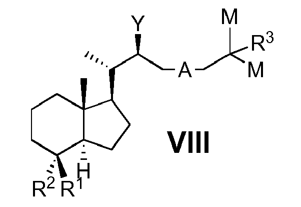 wherein
A is a single, double or triple bond,
M is a trideuteromethyl group,
Y is a hydroxyl, carbamate or diethylphosphate group,
R¹ is a hydroxyl, trialkylsilyloxy or benzoate group,
R² is hydrogen, or R¹ and R² can both be a halomethylene or carbonyl group.

12. The compounds of formula IX, X and XI 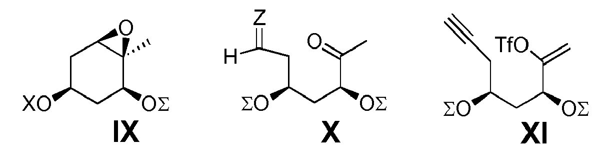 wherein
Z is an oxygen or a dibromomethylene group,
X is a hydrogen, a hydroxyl group or a trialkylsilyloxy group,
∑ is an SiR₃, SiR₂R', R and R' being alkyl or aryl radicals, or a methoxymethyl group.

13. Use of the compounds of formula I according to claim 1 in methods for the quantification of vitamin D, its metabolites and/or analogues by mass spectrometry.
